# EUROPEAN PATENT APPLICATION

(11) **EP 4 700 120 A1**
(43) Date of publication of application: **25.02.2026**
(21) Application number: 24792953.2
(22) Date of filing: 15.04.2024
(51) Int. Cl.: C12N 9/88, C12N 15/70, A23K 10/16

(54) **NOVEL POLYPEPTIDE WITH DON-DEGRADING ACTIVITY**

(30) Priority: 17.04.2023 KR 20230049989
(71) Applicant: CJ Cheiljedang Corporation, Seoul 04560 (KR)
(72) Inventor: KIM, Nam Yoon, Seoul 04560 (KR); LEE, Seung-hee, Seoul 04560 (KR); YANG, Tae Joo, Seoul 04560 (KR); HONG, So Yeon, Seoul 04560 (KR); KANG, Young Seo, Seoul 04560 (KR)
(74) Representative: Meissner Bolte Partnerschaft mbB
(86) International application number: PCT/KR2024/004993
(87) International publication number: WO 2024/219767

(57) **Abstract**

The present disclosure relates to a modified polypeptide having DON degradation activity.

## Description

### [Technical Field]

The present disclosure relates to a modified polypeptide having DON degradation activity.

### [Background Art]

Mycotoxins are a collective term for fungal-derived metabolites that are toxic to animals. When crops or stored agricultural products are infected or contaminated with fungi that produce mycotoxins, the generated mycotoxins can pose health risks to animals or humans. Trichothecene, which is often found in grains, is a sesquiterpenoid mycotoxin having a 12,13-epoxy-trichothec-9-ene structure and is mainly produced by *Fusarium, Myrothecium, Stachybotrys,* and *Trichothecium.* From an economic standpoint, the most important trichothecene-producing genus is *Fusarium,* and among these toxins, the ones most frequently detected in grains are deoxynivalenol (DON), nivalenol (NIV), and their acetylated derivatives.

Trichothecenes act on eukaryotic cells to exhibit various toxic effects such as inhibition of protein synthesis, cytotoxicity, and cell death. Ingestion of feed or food contaminated with DON can cause symptoms such as immunosuppression, anemia, headache, nausea, and abdominal pain in humans, and symptoms such as feed refusal, vomiting, growth inhibition, and reproductive disorders in animals. Due to the impact of global warming, the annual level of exposure of humans or animals to trichothecenes is expected to exceed tolerable levels.

Most countries have legally established allowable limits for DON detection in feed, food, and harvested grains. Attempts have been made to reduce DON contamination by using pesticides targeting *Fusarium* spp. or through breeding; however, DON still contaminates grains. To detoxify DON, various chemicals such as ozone, ammonia, chlorine, hydrogen peroxide, and sodium bisulfite have been used; however, these have not been scaled up due to cost, safety concerns, and adverse effects on grain quality. The most effective method is the treatment with sodium metabisulfite; however, in Europe, its application to food-use grains is prohibited, creating a need for a safe method of detoxifying DON.

### [Prior Art Documents]

### [Non-patent Documents]

(Non-patent document 1) S Chakraborty et al., Virulence. Jan 2015; 6(1): 50-65 (2014.12.17), Lactoylglutathione lyase, a critical enzyme in methylglyoxal detoxification, contributes to survival of Salmonella in the nutrient rich environment.

### [Disclosure]

### [Technical Problem]

The problem to be solved by the present disclosure is to provide a modified polypeptide having DON degradation activity.

### [Technical Solution]

An object of the present disclosure to provide a modified polypeptide having deoxynivalenol (DON) degradation activity, wherein the modified polypeptide is: 1) a polypeptide having an amino acid sequence of SEQ ID NO: 3; or 2) a polypeptide in which an amino acid at a position corresponding to position 65 of SEQ ID NO: 3 is substituted with a different amino acid.

Another object of the present disclosure is to provide a polynucleotide encoding the modified polypeptide.

Still another object of the present disclosure is to provide a microorganism comprising one or more of: the modified polypeptide; a polynucleotide encoding the modified polypeptide; and a vector comprising the polynucleotide.

Still another object of the present disclosure is to provide a composition for degrading DON, comprising one or more of: the modified polypeptide; and a microorganism comprising one or more of the modified polypeptide, a polynucleotide encoding the modified polypeptide, and a vector comprising the polynucleotide.

Still another object of the present disclosure is to provide a feed additive composition, comprising one or more of: the modified polypeptide; and a microorganism comprising one or more of the modified polypeptide, a polynucleotide encoding the modified polypeptide, and a vector comprising the polynucleotide.

Still another object of the present disclosure is to provide a method for degrading DON, comprising reacting DON with one or more of: the modified polypeptide; and a microorganism comprising one or more of the modified polypeptide, a polynucleotide encoding the modified polypeptide, and a vector comprising the polynucleotide.

Still another object of the present disclosure is to provide a method for preparing a feed product, comprising mixing a feed ingredient with a feed additive composition comprising one or more of: the modified polypeptide; and a microorganism comprising one or more of the modified polypeptide, a polynucleotide encoding the modified polypeptide, and a vector comprising the polynucleotide.

Still another object of the present disclosure is to provide a method for preparing a modified polypeptide having DON degradation activity, comprising culturing a microorganism comprising one or more of: the modified polypeptide, a polynucleotide encoding the modified polypeptide, and a vector comprising the polynucleotide.

### [Advantageous Effects]

The modified polypeptide having DON degradation activity of the present disclosure has improved thermal tolerance and thermal stability, and can be usefully applied in various industrial fields.

### [Brief Description of the Drawings]

FIG. 1 is a diagram confirming the thermal stability of a wild-type polypeptide (GhSPG-9; SEQ ID NO: 1) having DON degradation activity derived from *Gossypium hirsutum* and a modified polypeptide (SEQ ID NO: 3) having DON degradation activity of the present disclosure.
FIG. 2 is a diagram confirming the thermal stability of a wild-type polypeptide (GhSPG-9; SEQ ID NO: 1) having DON degradation activity derived from *Gossypium hirsutum* and modified polypeptides (SEQ ID NOS: 3 and 5) having DON degradation activity of the present disclosure.
FIG. 3 is a diagram confirming the effect of reduced cytotoxicity of a DON degradation product by the modified polypeptide having DON degradation activity of the present disclosure.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Furthermore, those skilled in the art may be able to recognize or identify numerous equivalents to the particular aspects of the present disclosure described herein by using routine experimentation. Moreover, these equivalents are intended to be included in the present disclosure.

Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

One aspect of the present disclosure provides a modified polypeptide having deoxynivalenol (DON) degradation activity, wherein the modified polypeptide is: 1) a polypeptide having an amino acid sequence of SEQ ID NO: 3; or 2) a polypeptide in which an amino acid at a position corresponding to position 65 of SEQ ID NO: 3 is substituted with a different amino acid.

As used herein, "deoxynivalenol degradation activity" means catalyzing the degradation of deoxynivalenol (DON) through conversion into the form of iso-DON.

As used herein, the polypeptide having DON degradation activity may be, for example, a glyoxalase I. In one example, it may be a glyoxalase SPG or a specialized glyoxalase (SPG), but is not limited thereto.

Meanwhile, the term "degradation" of DON (deoxynivalenol, DON) may be used to refer to detoxification of DON, inactivation of DON, and decontaminating DON-induced contamination.

Although the modified polypeptide having DON degradation activity provided herein is defined as: 1) a polypeptide having an amino acid sequence of SEQ ID NO: 3; or 2) a polypeptide in which an amino acid at a position corresponding to position 65 of SEQ ID NO: 3 is substituted with a different amino acid, this does not exclude additions of non-functional sequences upstream or downstream of the amino acid sequence of SEQ ID NO: 3, naturally occurring mutations, or silent mutations thereof. It is apparent to those skilled in the art that a protein having the same or corresponding activity as a protein consisting of the amino acid sequence of SEQ ID NO: 3 also falls within the scope of the modified polypeptide having DON degradation activity provided herein.

That is, even if the present disclosure describes a polypeptide or a protein "comprising" an amino acid sequence represented by a specific SEQ ID NO, a polypeptide or a protein "consisting of" an amino acid sequence represented by a specific SEQ ID NO, or a polypeptide or a protein "having" an amino acid sequence represented by a specific SEQ ID NO, it is apparent that a protein having an amino acid sequence with deletion, modification, substitution, or addition in part of the sequence may also be used in the present disclosure, as long as it has an activity identical or corresponding to that of the polypeptide or protein consisting of the amino acid sequence of the corresponding SEQ ID NO.

In addition, a polypeptide having DON degradation activity obtained by aligning the sequence of the modified polypeptide provided in the present disclosure with that of a wild-type DON-degrading polypeptide, comparing the sequence of the wild-type polypeptide having DON degradation activity with that of the modified polypeptide provided in the present disclosure, and introducing into the sequence of the wild-type polypeptide having DON degradation activity a modification corresponding to that of the modified polypeptide provided in the present disclosure also falls within the scope of the modified polypeptide provided in the present disclosure. Examples of such wild-type polypeptides having DON degradation activity include those derived from the genus *Gossypium, Handroanthus,* or *Hibiscus.* Specifically, a polypeptide having DON degradation activity derived from the genus *Gossypium* may be used. One example of the wild-type polypeptide having DON degradation activity may be a polypeptide having the amino acid sequence of SEQ ID NO: 1, or a polypeptide having at least 90%, 91%, 92%, 93%, 94%, 95%, 96%, 97%, 98%, or 99% or more identity thereto.

As used herein, the term "wild-type" refers to a naturally-occurring state without artificial modification. When the term "wild-type" is used in reference to a polypeptide, it refers to a naturally occurring polypeptide which does not have artificial mutations (substitutions, insertions, deletions, *etc*.) at one or more amino acid positions. Similarly, when the term "wild-type" is used in reference to a polynucleotide, it refers to a polynucleotide lacking artificial modifications (substitutions, insertions, deletions) of one or more nucleotides. However, polynucleotides encoding wild-type polypeptides are not limited to native polynucleotides and include sequences encoding any wild-type polypeptides.

The modified polypeptide provided herein can be referred to as a "modified polypeptide" or "variant" in that one or more amino acids are different compared to a wild-type polypeptide having DON degradation activity known in the art, for example, a wild-type polypeptide having DON degradation activity derived from the genus *Gossypium,* specifically the amino acid sequence of SEQ ID NO: 1.

In one embodiment, the modified polypeptide of the present disclosure may have 34 or 35 amino acid substitutions to different amino acids compared with the amino acid sequence of SEQ ID NO: 1, but is not limited thereto.

Further, in one embodiment, the modified polypeptide may have amino acid substitutions at 34 or 35 positions from positions 2, 4, 5, 6, 10, 17, 18, 28, 29, 41, 42, 44, 56, 57, 58, 62, 65, 69, 83, 85, 87, 96, 101, 106, 115, 121, 133, 142, 152, 172, 173, 176, 177, 180, and 181 of SEQ ID NO: 1, but is not limited thereto.

In one embodiment of the foregoing embodiments, the modified polypeptide provided herein may comprise the mutations G2A, L4S, D5G, S6P, P10A, H17Q, S18T, I28F, L29M, P41V, A42S, E44D, N56K, K57R, V58I, Y62D, M65F, M69F, V83I, K85R, I87A, M96I, F101K, N106S, D115N, T121R, M133T, S142R, G152D, N172K, G173N, A176N, I177V, N180G, and L181V of SEQ ID NO: 1, and may comprise SEQ ID NO: 3, but is not limited thereto.

The modified polypeptide provided herein may comprise not only SEQ ID NO: 3, but also a polypeptide in which the amino acid at the position corresponding to position 65 of SEQ ID NO: 3 is substituted with a different amino acid.

In one embodiment of the foregoing embodiments, the modified polypeptide provided herein may be one in which the amino acid at the position corresponding to position 65 of SEQ ID NO: 3 is G, A, V, L, I, M, F, W, P, S, T, C, Y, N, Q, D, E, K, R, or H. Specifically, it may be M.

In one embodiment of the foregoing embodiments, the modified polypeptide provided herein may be one in which the amino acid at the position corresponding to position 65 of SEQ ID NO: 3 is substituted with a nonpolar amino acid.

The modified polypeptide may be one in which the amino acid at the position corresponding to position 65 of SEQ ID NO: 3 is substituted with methionine (M), but is not limited thereto.

The modified polypeptide may comprise SEQ ID NO: 5.

As used herein, the term "corresponding to" refers to an amino acid residue at the position recited in a protein or peptide, or an amino acid residue that is similar, identical, or homologous to the residue recited in a protein or peptide. Identification of the amino acid at the corresponding position may be determining a specific amino acid in a sequence that references a particular sequence. As used herein, the term "corresponding region" refers to a similar or corresponding position in a related protein or a reference protein.

In the present disclosure, SEQ ID NO: 1 may be used as a reference sequence to determine the position of amino acids in any amino acid sequence.

That is, SEQ ID NO: 1 disclosed herein can be used to determine the corresponding amino acid residues in any polypeptide having DON degradation activity. Unless indicated otherwise in the present disclosure, residues of a specific amino acid sequence are numbered relative to SEQ ID NO: 1.

For example, based on the alignment of any amino acid sequence with SEQ ID NO: 1, each amino acid residue in the amino acid sequence can be numbered with reference to the position number of the amino acid residue corresponding to the amino acid residue of SEQ ID NO: 1. For example, a sequence alignment algorithm as described herein can identify the position of an amino acid or a position where modification such as substitution, insertion, or deletion occurs compared to a query sequence (also referred to as the "reference sequence").

An example of the alignment may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277), *etc.,* but the alignment is not limited thereto.

In addition, multiple sequence alignment can be used to identify corresponding amino acid residues in other polypeptides having DON degradation activity. Examples of the multiple sequence alignment known in the art include programs such as MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680) using their respective default parameters, but are not limited thereto.

In addition, when the relationship between enzymes diverged from a mature polypeptide of SEQ ID NO: 1 cannot be identified by traditional sequence-based comparison, other pairwise sequence comparison algorithms may be employed (Lindahl and Elofsson, 2000, J. Mol. Biol. 295: 613-615). Higher sensitivity can be achieved in sequence-based searches by using search programs that utilize probabilistic representations of polypeptide families (profiles) to search databases. For example, the PSI BLAST program can generate profiles through an iterative database search process and detect remote homologs (Atschul et al., 1997, Nucleic Acids Res. 25: 3389-3402). When the family or superfamily of a polypeptide has one or more representatives in protein structure databases, significantly higher sensitivity can be achieved. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be sequentially used to generate homology models for the peptides, and such models can be assessed for accuracy using a variety of tools developed for that purpose.

For proteins of known structure, several tools and resources may be used for retrieving and generating structural alignments. For example, the SCOP superfamilies of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747), and implementation of these algorithms can additionally be utilized to query structure databases with a structure of interest in order to discover possible structural homologs (Holm and Park, 2000, Bioinformatics 16: 566-567).

The foregoing methods are illustrative examples and are not limited thereto.

Throughout the entire specification of the present disclosure, the conventional one-letter and three-letter codes for naturally occurring amino acids are used. Additionally, the amino acids mentioned herein are abbreviated according to the IUPAC-IUB nomenclature as follows:

| | |
|---|---|
| Alanine Ala, A | Arginine Arg, R |
| Asparagine Asn, N | Aspartic acid Asp, D |
| Cysteine Cys, C | Glutamic acid Glu, E |
| Glutamine Gln, Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

Meanwhile, any amino acid may be described as Xaa or X.

In addition to naturally occurring amino acids, commonly accepted three-letter codes may also be used for other amino acids such as 2-aminoisobutyric acid (Aib), N-methylglycine (Sar), and α-methyl-glutamic acid.

Amino acids can generally be classified based on similarity in polarity, charge, solubility, hydrophobicity, hydrophilicity, and/or amphipathic nature of the residues. Accordingly, amino acid substitution may generally occur based on similarity in polarity, charge, solubility, hydrophobicity, hydrophilicity, and/or amphipathic nature of the residues.

For example, among the amino acids having an electrically charged side chain (electrically charged amino acids), positively charged (basic) amino acids include arginine, lysine, and histidine, and negatively charged (acidic) amino acids include glutamic acid and aspartic acid. Further, among the amino acids having an uncharged side chain (uncharged amino acids), nonpolar amino acids include glycine, alanine, valine, leucine, isoleucine, methionine, phenylalanine, tryptophan, and proline, and polar or hydrophilic amino acids include serine, threonine, cysteine, tyrosine, asparagine, and glutamine. Among the nonpolar amino acids, aromatic amino acids include phenylalanine, tryptophan, and tyrosine.

In addition, polypeptides in which one or more amino acids differ from the recited sequence by conservative substitution and/or modification relative to the amino acid sequence of SEQ ID NO: 3 or SEQ ID NO: 5, but in which the functions or properties of the protein are maintained, are also included within the scope of the modified polypeptide provided herein. Such modifications may, for example, include removal of a portion from the N- and/or C-terminus of a mature protein.

As used herein, the term "conservative substitution" refers to the substitution of an amino acid with another amino acid having similar structural and/or chemical properties. The modified polypeptide of the present disclosure may have, for example, one or more conservative constitutions, while still retaining one or more biological activities of the modified polypeptide of SEQ ID NO: 3 or SEQ ID NO: 5. Such an amino acid substitution may generally occur based on similarity in polarity, charge, solubility, hydrophobicity, hydrophilicity, and/or amphipathic nature of the residues.

In one embodiment, the modified polypeptide having DON degradation activity provided herein may comprise 1) an amino acid sequence of SEQ ID NO: 3 or 2) a modified polypeptide in which the amino acid at the position corresponding to position 65 of SEQ ID NO: 3, comprise a modified polypeptide of SEQ ID NO: 3 or SEQ ID NO: 5, consist essentially of SEQ ID NO: 3 or SEQ ID NO: 5, or consist of SEQ ID NO: 3 or SEQ ID NO: 5. In another embodiment, the modified polypeptide having DON degradation activity provided herein may be a polypeptide having at least 80%, 90%, 91%, 92%, 93%, 94%, 95%, 96%, 97%, 98%, 99%, or 100% homology or identity to SEQ ID NO: 3 or SEQ ID NO: 5, or comprises, consists essentially of, or consists of a sequence having such homology or identity.

As used herein, the term "homology" or "identity" refers to the degree of relevance between two given amino acid sequences or nucleotide sequences, and may be expressed as a percentage. The terms "homology" and "identity" may often be used interchangeably.

The sequence homology or identity of conserved polynucleotides or polypeptides may be determined by a standard alignment algorithm, and default gap penalties established by the program used may be applied. Substantially, homologous or identical sequences may generally hybridize under moderately or highly stringent conditions to the full-length of the sequence or at least about 50%, 60%, 70%, 80%, or 90% or more of the full-length. It is apparent that hybridization also includes hybridization with polynucleotides containing codons that reflect common codon usage or codon degeneracy in the polynucleotides.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)) , BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, the homology, similarity, or identity may be determined by using BLAST of the National Center for Biotechnology Information or ClustalW.

The homology, similarity, or identity of polynucleotides or polypeptides may be determined by comparing sequence information using, for example, the GAP computer program, such as Needleman et al. (1970), J Mol Biol. 48:443, as disclosed in Smith and Waterman, Adv. Appl. Math (1981) 2:482. In summary, the GAP program may be defined as the value obtained by dividing the number of similarly aligned symbols (*i.e.,* nucleotides or amino acids) by the total number of the symbols in the shorter of the two sequences. The default parameters for the GAP program may include: (1) a binary comparison matrix (containing a value of 1 for identity and 0 for non- identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

Further, whether any two polynucleotide or polypeptide sequences have homology, similarity, or identity may be identified by comparing the sequences in a Southern hybridization experiment under stringent conditions as defined. Appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). However, the method for identifying homology, similarity, or identity and the appropriate hybridization conditions are not limited thereto.

As used herein, "enzymatic activity" or "DON degradation activity" represents at least one catalytic activity. Specifically, it may be the conversion efficiency of an enzyme mainly expressed as k_{cat}/Km, but is not limited thereto.

k_{cat} refers to the catalytic constant for the rate at which a single enzyme converts substrates into products per unit time when the enzyme is fully saturated with the substrates, and is also called the turnover number. Km refers to the substrate concentration at which the reaction rate is at half the maximum value (Vmax).

Examples of the methods of expressing enzymatic activity include specific activity (µmol of converted substrate x mg ⁻¹ x min ⁻¹) or volumetric activity (µmol of converted substrate x mL ⁻¹ x min ⁻¹). However, the definition of enzymatic activity is not limited to the description above and may be defined and evaluated based on the information known in the art.

As used herein, enzyme stability means that enzyme activity is retained during storage or reaction time. In order to measure such changes in stability, the initial enzymatic activity can be measured and compared under defined conditions at time zero (100%) and after a certain period of time (x %), and thus, the level at which the enzymatic activity is lost or enzyme stability can be expressed.

Factors that affect enzymatic activity include, for example, pH, heat, the presence of other substances (*e.g*., oxidizing agents, chelating agents), *etc.*

As used herein, the term "thermal stability" refers to the ability of a protein to function in a specific temperature range. In one embodiment, the polypeptide provided herein may be active in a range of about 20°C to about 120°C, and specifically in a range of about 60°C to about 100°C. In any one embodiment of the foregoing embodiments, the polypeptide having DON degradation activity provided herein may be active at 50°C to 80°C, but the activity is not limited thereto.

As used herein, the term "thermal tolerance" refers to the ability of a protein to function after exposure to a specific temperature, *e.g*., high heat or cryogenic temperature. For example, proteins with thermal tolerance may not function at non-optimal temperatures, but may regain their function upon returning to optimal temperature conditions.

Increased stability includes an expanded range of pH, temperature, and/or duration, *etc.,* in which a protein retains its function or maintains high enzymatic activity, as compared to other enzymes such as wild-type enzymes, parent enzymes, and/or other modified polypeptides. In the present disclosure, increased stability may be evaluated relative to a wild-type DON-degrading polypeptide, for example, the polypeptide of SEQ ID NO: 1 having DON degradation activity, but is not limited thereto.

In one embodiment, the modified polypeptide having DON degradation activity provided herein may have one or more improved properties selected from thermal tolerance and thermal stability relative to those of the polypeptide of SEQ ID NO: 1.

In any one embodiment of the foregoing embodiments, the modified polypeptide may retain 20% or more relative activity at 50°C to 80°C; but the relative activity is not limited thereto.

In any one embodiment of the foregoing embodiments, the properties of the modified polypeptide having DON degradation activity provided herein may be activities or improved activities suitable for applications in various industries including feed, baking, and pulp bleaching.

Another aspect of the present disclosure provides a polynucleotide encoding the modified polypeptide having DON degradation activity of the present disclosure.

As used herein, the term "polynucleotide" refers to a DNA or RNA strand of at least a certain length and is a polymer of nucleotides, in which nucleotide monomers are connected in a long chain by covalent bonds.

The polynucleotide encoding the modified polypeptide having DON degradation activity of the present disclosure includes, without limitation, any polynucleotide as long as it encodes: the modified polypeptide, the polypeptide of SEQ ID NO: 3 or SEQ ID NO: 5, or a polypeptide having corresponding activity thereto.

The polynucleotide encoding the modified polypeptide having DON degradation activity of the present disclosure may undergo various modifications in the coding region within the scope that does not change the amino acid sequence of the polypeptide, due to codon degeneracy or in consideration of the codons preferred in an organism in which the modified polypeptide is to be expressed.

For example, the polynucleotide encoding the modified polypeptide having DON degradation activity of the present disclosure may be a polynucleotide sequence encoding the amino acid sequence of SEQ ID NO: 3 or SEQ ID NO: 5, or a polypeptide having homology or identity thereto.

In one embodiment, the polynucleotide encoding the modified polypeptide having DON degradation activity of the present disclosure may have or comprise a nucleotide sequence having at least 50%, 60%, 70%, 75%, 80%, 85%, or 90% and less than 100% homology or identity to the polynucleotide sequence of SEQ ID NO: 2. In another embodiment, the polynucleotide encoding the modified polypeptide having DON degradation activity of the present disclosure may consist of or consist essentially of a nucleotide sequence having at least 70%, 75%, 80%, 85%, 90%, 95%, 96%, 97%, 98%, or 99% homology or identity to the sequence of SEQ ID NO: 4 or SEQ ID NO: 6. Additionally, the polynucleotide of the present disclosure may include, without limitation, a probe that can be prepared from a known gene sequence, for example, any polynucleotide sequence which may hybridize with a sequence complementary to all or part of the polynucleotide sequence of the present disclosure under stringent conditions.

The term "stringent conditions" refers to conditions that enable specific hybridization between polynucleotides. Such conditions are disclosed in detail in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, i.e., polynucleotides having 70% or more, 75% or more, 80% or more, 85% or more, 90% or more, 95% or more, 96% or more, 97% or more, 98% or more, or 99% or more homology or identity hybridize with each other while polynucleotides having homology or identity lower than the above-mentioned homology or identity do not hybridize with each other, or may include ordinary washing conditions of Southern hybridization, *i.e.*, washing once, specifically twice or three times, at a salt concentration and temperature corresponding to 60°C., 1×SSC, 0.1% SDS, specifically 60°C., 0.1×SSC, 0.1% SDS, and more specifically 68°C., 0.1×SSC, 0.1% SDS.

Hybridization requires that two nucleic acids have complementary sequences, although base mismatches are permissible depending on the stringency of the hybridization. The term "complementary" is used to describe a relationship between nucleotide bases that can hybridize with each other. For example, regarding DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having homology or identity to the polynucleotide of the present disclosure may be detected using the hybridization conditions including a hybridization step at a Tₘ value of 55 °C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (e.g., Sambrook *et al.,* supra).

Still another aspect of the present disclosure provides a vector comprising the polynucleotide encoding the modified polypeptide having DON degradation activity of the present disclosure. The modified polypeptide and polynucleotide are as described in the aspects above.

As used herein, the term "vector" refers to a DNA construct containing the nucleotide sequence of a polynucleotide encoding the target polypeptide, operably linked to an appropriate expression control region (or expression control sequence) so as to express the target polypeptide in an appropriate host. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

For example, a polynucleotide encoding a target polypeptide within a chromosome may be replaced with a modified polynucleotide through a vector for chromosomal insertion in a cell. The insertion of the polynucleotide into the chromosome may be performed by any method known in the art, such as homologous recombination, but is not limited thereto. A selection marker for confirming the chromosomal insertion may be further included. The selection marker is used to screen cells transformed via the vector, that is, to confirm the insertion of the desired nucleic acid molecule. Markers that confer selectable phenotypes, such as drug resistance, nutrient requirements, resistance to cytotoxic agents, or expression of surface proteins, may be used. In an environment treated with a selective agent, only the cells that express the selection marker survive or exhibit a distinct phenotype, allowing for the selection of transformed cells.

The vector used in the present disclosure is not particularly limited, and any vector known in the art may be used.

Examples of commonly used vectors in prokaryotic cells, as a phage vector or cosmid vector, pWE15, M13, MBL3, MBL4, IXII, ASHII, APII, t10, t11, Charon4A, and Charon21A, *etc.* may be used, and as a plasmid vector, the pBR series, pUC series, pBluescriptll series, pGEM series, pTZ series, pCL series, and pET series, etc. may be used. Specifically, vectors including pDZ, pACYC177, pACYC184, pCL, pECCG117, pUC19, pBR322, pMW118, pCC1BAC, and pDCM2 may be used.

As an example of a vector used in eukaryotic cells, a yeast expression vector may be an integrative yeast plasmid (YIp) or an extrachromosomal plasmid vector. The extrachromosomal plasmid vector may comprise a yeast episomal plasmid (YEp), a yeast replicative plasmid (YRp), or a yeast centromere plasmid (YCp). In addition, artificial yeast chromosomes (YACs) may also be used as the vector of the present disclosure. Specific examples include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405, and pYJ406, but are not limited thereto.

As used herein, the term "transformation" refers to the introduction of a vector containing a polynucleotide encoding a target protein into a host cell or a microorganism to enable the expression of the protein encoded by the polynucleotide within the host cell. As long as the transformed polynucleotide can be expressed within the host cell, the transformed polynucleotide may be located within the chromosome of the host cell or exist extrachromosomally. Further, the polynucleotide comprises DNA and RNA encoding the target protein. The polynucleotide may be introduced in any form, provided that it can be introduced into and expressed within a host cell. For example, the polynucleotide may be introduced into a host cell in the form of an expression cassette, which is a gene construct comprising all elements necessary for self-expression. The expression cassette may comprise a promoter operably linked to the polynucleotide, a transcription termination signal, a ribosome binding site, and a translation termination signal. The expression cassette may be in the form of a self-replicating expression vector. Further, the polynucleotide may be introduced into a host cell as-is and operably linked to a sequence necessary for expression in the host cell, but is not limited thereto.

Additionally, the term "operably linked" used herein refers to a functional linkage between a promoter sequence for initiating and mediating the transcription of the polynucleotide encoding the target polypeptide of the present disclosure, and the polynucleotide sequence.

The method for transforming the vector of the present disclosure includes any method of introducing a nucleic acid into a cell and may be performed by selecting a suitable standard technique as known in the art depending on the host cell. For example, the transformation may be carried out via electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but the method is not limited thereto.

Still another aspect of the present disclosure provides a microorganism comprising one or more of: the modified polypeptide; a polynucleotide encoding the modified polypeptide; and a vector comprising the polynucleotide.

The microorganism may comprise the modified polypeptide, the polynucleotide encoding the same, a nucleic acid construct comprising the polynucleotide and/or a vector comprising the polynucleotide. The nucleic acid construct or vector may be integrated into the chromosome or may be maintained as a self-replicating extrachromosomal vector.

The microorganism of the present disclosure may include any microorganism without limitation, as long as it can express the modified polypeptide having DON degradation activity of the present disclosure. For example, the microorganism includes any cell useful in the recombinant production of a modified polypeptide having DON degradation activity of the present disclosure, for example, a prokaryotic cell or a eukaryotic cell. In another example, examples of the microorganism include fungi and bacteria.

In one embodiment, the microorganism may be of the genus *Escherichia,* for example *Escherichia coli, Escherichia albertii, Escherichia fergusonii, Escherichia hermannii, Escherichia vulneris,* or *Escherichia blattae.* In any any one embodiment of the preceding embodiments, the microorganism may be *Escherichia coli,* but is not limited thereto.

Still another aspect of the present disclosure provides a composition for degrading DON, comprising one or more of: the modified polypeptide having DON degradation activity of the present disclosure; and a microorganism comprising one or more of the modified polypeptide, a polynucleotide encoding the modified polypeptide, and a vector comprising the polynucleotide.

Still another aspect of the present disclosure provides a method for degrading DON, comprising reacting DON with one or more of: the modified polypeptide having DON degradation activity of the present disclosure; and a microorganism comprising one or more of the modified polypeptide, a polynucleotide encoding the modified polypeptide, and a vector comprising the polynucleotide.

The modified polypeptide having DON degradation activity of the present disclosure and/or the microorganism comprising one or more of the modified polypeptide, a polynucleotide encoding the modified polypeptide, and a vector comprising the polynucleotide may be used to degrade substances containing DON.

As used herein, deoxynivalenol (DON) has the chemical formula C₁₅H₂₀O₆ and a molecular weight of 296.3 g/mol (CAS No. 51481-10-8). It is a fungal toxin belonging to type B trichothecenes, which is an epoxy-sesquiterpenoid, and is also known as vomitoxin. It can be produced by fungi that cause plant diseases, among which species of the genus *Fusarium* are known producers. Upon ingestion, DON can cause symptoms such as immunosuppression, anemia, headache, nausea, and abdominal pain in humans, and feed refusal, vomiting, growth inhibition, and reproductive disorders in animals.

In one embodiment, the polypeptide of the present disclosure may be used for detoxification of DON, detoxification of toxicity of DON, conversion of DON to iso-DON, and/or degradation induction through conversion of iso-DON.

Additionally, in another embodiment of the present disclosure, the polypeptide of the present disclosure may be used in the detoxification, decontamination, or degradation of toxicity (e.g. degradation) of food contaminated with DON.

In one embodiment, the composition for degrading DON provided herein may further comprise a naturally occurring substance or a non-naturally occurring substance, in addition to the modified polypeptide of the present disclosure.

In one embodiment, the composition for degrading DON provided herein may further comprise any component suitable for applications in various industries including feed, baking, biomass saccharification, and pulp bleaching.

Examples of substances that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, *etc.,* but are not limited thereto.

Still another aspect of the present disclosure provides a feed additive composition comprising one or more of: the modified polypeptide having DON degradation activity of the present disclosure; and a microorganism comprising one or more of the modified polypeptide, a polynucleotide encoding the modified polypeptide, and a vector comprising the polynucleotide.

Still another aspect of the present disclosure provides a method for preparing a feed product, comprising mixing a feed ingredient with a feed additive composition comprising one or more of: the modified polypeptide having DON degradation activity of the present disclosure; and a microorganism comprising one or more of the modified polypeptide, a polynucleotide encoding the modified polypeptide, and a vector comprising the polynucleotide.

The microorganism comprising the modified polypeptide, a polynucleotide encoding the modified polypeptide, and/or a vector comprising the polynucleotide may be used for any one of the following applications:
a) additives in animal feed ingredients; and/or
b) animal feed supplements; and/or
c) degradation of grain-based materials (*e.g.,* whole grains or portions thereof).

The feed product of the present disclosure may refer to any natural or artificial diet, meal, or an ingredient of the meal that is intended for or suitable for consumption, ingestion, and digestion by animals, and can be prepared in various forms known in the art.

In one embodiment, the feed product may be a grain-based material (whole or partial grains or malt grains, *e.g*., wheat, barley, corn, oats, rye, rice, sorghum, *etc*.).

Still another aspect of the present disclosure provides a method for preparing a modified polypeptide having DON degradation activity, comprising culturing a microorganism comprising one or more of: the modified polypeptide having DON degradation activity of the present disclosure, a polynucleotide encoding the modified polypeptide, and a vector comprising the polynucleotide.

The culturing may include culturing the microorganism in a culture medium.

As used herein, the term "culturing" means that the host cell is grown under appropriately controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, or fed-batch culturing, but is not limited thereto.

As used herein, the term "medium" refers to a mixed substance containing nutrients required to culture the host cell as a main component, and the medium supplies nutrients, growth factors, *etc.,* including water, which are indispensable for survival and development. Specifically, any medium and culture conditions may be used for culturing the host cell without particular limitation, as long as the medium is used for the common culture of host cells. The host cell may be cultured in a common medium containing suitable carbon sources, nitrogen sources, phosphorus sources, inorganic compounds, amino acids, and/or vitamins, *etc*., while controlling the temperature, pH, *etc*. under aerobic conditions.

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e*., molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monobasic phosphate, dibasic potassium phosphate, or the corresponding sodium-containing salts may be used. As the inorganic compounds, sodium chloride, calcium chloride, iron chloride, magnesium sulfate, iron sulfate, manganese sulfate, calcium carbonate, *etc.* may be used. In addition, amino acids, vitamins, and/or suitable precursors, *etc.* may be included. These components or precursors may be added to the medium in a batchwise or continuous manner. However, the medium is not limited thereto.

During the culturing of the host cell, compounds such as ammonium hydroxide, potassium hydroxide, ammonia, phosphoric acid, and sulfuric acid may be added to the medium in an appropriate manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain anaerobic or microaerobic state of the medium, no gas or nitrogen, hydrogen, or carbon dioxide gas may be injected. However, the culturing conditions are not limited thereto
The temperature during the culturing of the present disclosure may be in the range from 20°C to 55°C, specifically from 25°C to 40°C, but is not limited thereto. The culturing may be carried out until a desired amount of a useful substance is obtained, and may be specifically carried out for 24 to 196 hours, but is not limited thereto.

In one embodiment, the method for preparing a modified polypeptide having DON degradation activity of the present disclosure may further comprise recovering the modified polypeptide having DON degradation activity of the present disclosure expressed during the culturing.

In another embodiment, the modified polypeptide having DON degradation activity expressed during the culturing may be recovered using a method known in the field to which the present invention pertains. For example, the modified polypeptide may be recovered from the medium through a conventional procedure including, but not limited to, collection, centrifugation, filtration, extraction, spray-drying, evaporation or precipitation.

The recovery method may be collecting the polypeptide using suitable methods known in the art depending on the culturing method of the microorganism of the present disclosure, such as batch, continuous, or fed-batch culturing methods. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC and a combination thereof may be used, and the modified polypeptide of the present disclosure can be recovered from the medium or the host cell using suitable methods known in the art.

In another embodiment, the modified polypeptide expressed by the host cell in the culturing step may not be recovered. In the foregoing embodiment, the host cell expressing the modified polypeptide may itself be used as a source of the modified polypeptide.

Still another aspect of the present disclosure provides a use of one or more of: the modified polypeptide of the present disclosure; a microorganism comprising one or more of the modified polypeptide, a polynucleotide encoding the modified polypeptide, and a vector comprising the polynucleotide, for degrading DON.

The modified polypeptide, polynucleotide, vector, microorganism, and DON degradation is as described above.

### [Mode for Carrying Out the Invention]

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

### Example 1: Preparation of Modified Polypeptide (GhSPG-9 Mutant) Having DON Degradation Activity

### Example 1-1. Construction of GhSPG-9 and Multiple Mutant

Polynucleotides (SEQ ID NOS: 2 and 4) encoding specialized glyoxalase (SPG) (hereinafter referred to as GhSPG-9; SEQ ID NO: 1) derived from *Gossypium harknessii* and a mutant (hereinafter referred to as GhSPG-216; SEQ ID NO: 3) in which 35 mutations were introduced into the GhSPG-9 sequence to improve thermal stability were synthesized by Cosmo Genetech, cloned into pET vectors (Novagen), and used to construct expression vectors.

### Example 1-2. Construction of GhSPG-216 Point Mutant

To improve the thermal stability of GhSPG-216, mutation sites were selected, and primers were designed to construct a point mutant (hereinafter referred to as GhSPG-216M1; SEQ ID NO: 5). Mutation position based on the amino acid sequence of SEQ ID NO: 5, amino acid after mutation, and primer sequences for mutant construction are disclosed in Table 1 below in the listed order.

**[Table 1]**

| **Mutation** | **SEQ ID NO** | **Name** | **Primer sequence (5'- 3')** |
|---|---|---|---|
| F65M | 7 | GhSPG-216M1-F | CCCGGATATGAAGATGACGTTATATTTTATGG GATATGAAG |
| | 8 | GhSPG-216M1-R | ACGTCATCTTCATATCCGGGACGTCAATTCTTT TCAAAAG |

Specifically, a GhSPG-9 point mutant was constructed by PCR using, as a template, the polynucleotide encoding GhSPG-216 (SEQ ID NO: 3) constructed in Example 1-1 cloned into a pET vector, and by using primers (SEQ ID NOS: 7 and 8 in Table 1) and a PCR premix (iNtRON, cat. no. 25185). PCR was carried out using an Eppendorf Mastercycler Nexus GX2, and the reaction conditions were as follows:
initial denaturation at 94°C for 2 minutes;
denaturation at 94°C for 20 seconds,
annealing at 50°C for 10 seconds, and
extension at 72°C for 8 minutes (15 cycles of denaturation to extension); and
final extension at 72°C for 5 minutes.

The generated truncated mutant was ligated using a QuickChange Site-Directed Mutagenesis kit (Agilent, cat. #200518), and then transformed into *E*. *coli* DH5α strain to confirm sequence mutations through sequencing.

### Example 2: Confirmation of Improved Thermal Stability of Modified Polypeptide (GhSPG-9 Mutant) Having DON Degradation Activity

### Example 2-1. Thermal Stability of GhSPG-9 Multiple Mutant Enzyme

GhSPG-9 and the GhSPG-216 mutants constructed in Example 1 were transformed into E. *coli* BL21(DE3), which were then inoculated into sterilized LB medium (BD Difco) and pre-cultured at 37°C and 200 rpm for 16 hours. Thereafter, 1/100 of the culture volume was inoculated into a flask containing sterile LB medium and cultured at 37°C and 200 rpm to an OD₆₀₀ of 0.4 to 0.5. Isopropyl β-D-1-thiogalactopyranoside (IPTG) was then added to a final concentration of 1 mM, cultured for an additional 16 hours, and the cells were recovered by centrifugation. The recovered cells were added to and resuspended in 20 mL of lysis buffer (50 mM Tris-HCl pH 8.0, 100 mM NaCl, 10 mM imidazole), and a crude enzyme solution was obtained by sonication and centrifugation. The crude enzyme solution was slowly poured onto Ni-NTA resin (Qiagen, cat. no. 30230) for adsorption, which was then washed with wash buffer (same as lysis buffer except 20 mM imidazole) and eluted with elution buffer (same as lysis buffer except 250 mM imidazole) to purify the enzyme.

Protein concentration was determined by mixing 5 µL of the diluted enzyme solution with 250 µL of Bradford solution (Quick Start^{™} Bradford 1× Dye Reagent, #5000205) and measuring the absorbance at 595 nm.

To analyze the activities of purified GhSPG-9 and GhSPG-216 mutants for DON, DON (CAS 51481-10-8) dissolved in distilled water and a purified enzyme reaction solution (25 mM Tris-HCl, 150 mM NaCl, pH 7.4) were prepared. After treatment with 500 µM of NiCl₂, the reaction was carried out at 50°C for 24 hours and incubated at 100°C for 5 minutes to terminate the reaction. The amount of residual DON in the reaction mixture was measured using High-Performance Liquid Chromatography-Ultraviolet (HPLC-UV), and the ratio of DON degradation was calculated to determine the titer.

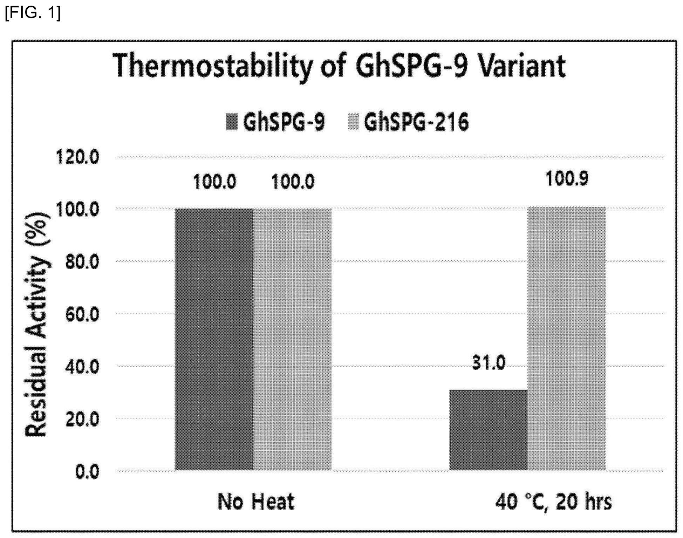

To evaluate the thermal stability (residual activity) of GhSPG-9 and GhSPG-216 mutants, 50 µL of each purified enzyme was incubated at 40°C for 20 hours. Using enzyme solutions before and after heat treatment, the amount of residual DON and the degradation ratio after the DON degradation reaction were determined, which are shown in FIG. 1. The results are as shown in Table 2.

**[Table 2]**

| **Mutant** | **DON Remaining (%)** | | **DON Degradation (%)** | | **Residual Activity (%)** |
|---|---|---|---|---|---|
| | **No Heat** | **40°C, 20 hrs** | **No Heat** | **40°C, 20 hrs** | **40°C, 20 hrs** |
| GhSPG-9 | 8.9 | 71.8 | 91 .2 | 28.3 | 31.0 |
| GhSPG-216 | 12.5 | 11.7 | 87.5 | 88 .3 | 100.9 |

As a result, after incubation at 40°C for 20 hours, it was confirmed that GhSPG-9 retained 28% activity, whereas GhSPG-216 retained 100% activity.

### Example 2-2. Thermal Stability of GhSPG-216 Single Mutant Enzyme

The GhSPG-216M1 mutant constructed in Example 1 was transformed into E. *coli* BL21(DE3), and after expression, purification, and reaction of the enzyme by methods described in Example 2-1, reaction products were analyzed using HPLC-UV.

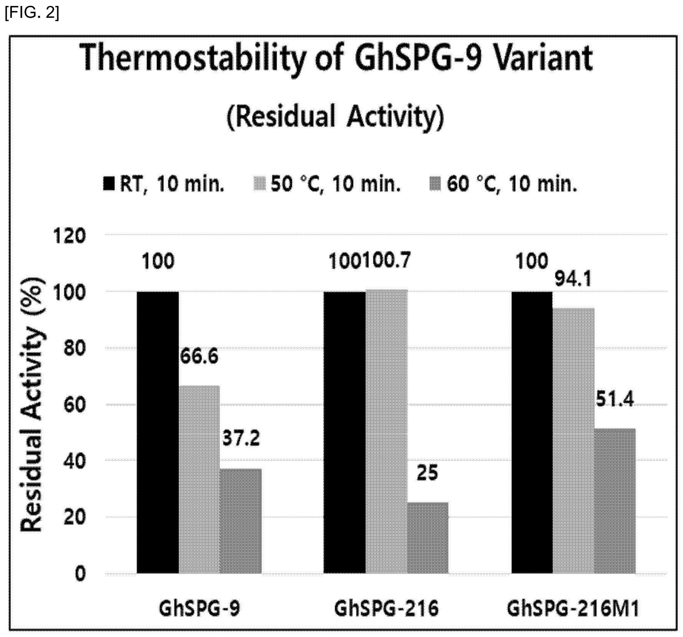

To evaluate the thermal stability (residual activity) of GhSPG-9, GhSPG-216, and GhSPG-216M1 mutant, 50 µL of each purified enzyme was incubated at room temperature, 50°C, and 60°C for 10 minutes. Using enzyme solutions before and after heat treatment, the amount of residual DON, the degradation ratio, and the residual activity after the DON degradation reaction were determined and are shown in FIG. 2. The results are as shown in Table 3.

**[Table 3]**

| **Mutant** | **DON Remaining (%)** | | | **DON Degradation (%)** | | | **Residual Activity (%)** | | |
|---|---|---|---|---|---|---|---|---|---|
| | **RT, 10 min.** | **50°C, 10 min.** | **60°C, 10 min.** | **RT, 10 min.** | **50°C, 10 min.** | **60°C, 10 min.** | **RT, 10 min.** | **50°C, 10 min.** | **60°C, 10 min.** |
| **GhSPG -9** | 44.6 | 63.1 | 79.4 | 55.4 | 36.9 | 20.6 | 100.0 | 66.6 | 37.2 |
| **GhSPG -216** | 76.6 | 76.4 | 94.2 | 23.4 | 23.6 | 5.8 | 100.0 | 100.7 | 25.0 |
| **GhSPG -216M1** | 73.1 | 74.7 | 86 .2 | 26.9 | 25.3 | 13.8 | 100.0 | 94.1 | 51.4 |

As a result, upon incubation at 50°C for 10 minutes, the residual activities of GhSPG-9, GhSPG-216, and GhSPG-216M1 relative to pre-treatment were 66.6%, 100.7%, and 94.1%, respectively, indicating that the residual activity of the point mutant GhSPG-216M1 was similar to that of its template GhSPG-216. Further, upon incubation at 60°C for 10 minutes, the residual activities of GhSPG-9, GhSPG-216, and GhSPG-216M1 were 37.2%, 25.0%, and 51.4%, respectively, confirming that thermal stability of GhSPG-216M1 was improved through point mutation.

### Example 3: Confirmation of the Effect of Reduced Cytotoxicity of DON Degradation Products by Modified Polypeptide (GhSPG-216) Having DON Degradation Activity

### Example 3-1. Evaluation of Cytotoxicity of DON Degradation Products by Enzyme GhSPG-216

Using the *E*. *coli* BL21(DE3) strain transformed with the GhSPG-216 expression vector constructed in Example 1, the enzyme was purified as described in Example 2. Protein concentration was determined by mixing 5 µL of the diluted enzyme solution with 250 µL of Bradford solution (Quick Start^{™} Bradford 1× Dye Reagent, #5000205) and measuring the absorbance at 595 nm.

To evaluate the cytotoxicity of DON degradation products by GhSPG-216, reaction of three samples, DON control, DON + GhSPG-216, and DON + inactivated GhSPG-216, were carried out for 48 hours under the same reaction conditions as described in Example 2. The concentration of DON in each sample was confirmed by HPLC-UV based on the peak area at 218 nm, and it was confirmed that DON was degraded to a level of about 95%. The detailed results are as shown in Table 4.

**[Table 4]**

| **Sample** | **Enzyme State** | **LC-UV Peak Area (218 nm)** | **% DON Remaining** |
|---|---|---|---|
| DON Control | N/A | 231.63 | 100.00 |
| DON + Inactive | Inactive | 235.91 | 101.85 |
| DON + GhSPG-216 | Active | 11.09 | 4.79 |

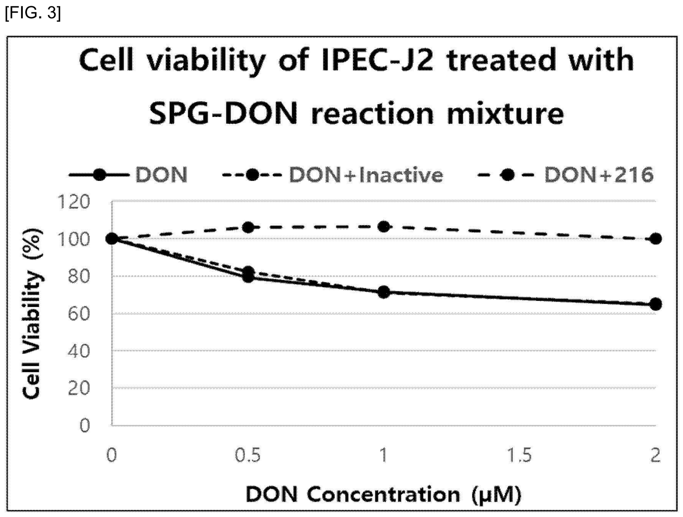

A DMEM/F-12 medium supplemented with 10% FBS and 1% penicillin-streptomycin was added to a T75 flask, and the IPEC-J2 cell line was subcultured in the flask at 37°C and under 5% CO₂. For cytotoxicity evaluation, the cultured cells were seeded in 96-well plates containing DMEM/F-12 with 10% FBS to 3×10⁴ cells/100 µL, and the cells were incubated for 24 hour under the above conditions. Thereafter, the existing medium was removed, and the three samples in Table 4 were each applied to the cells so as to final concentrations of 2 µM, 1 µM, and 0.5 µM (based on the substrate concentration before the reaction), followed by additional incubation at 37°C in a CO₂ incubator for 24 hours. After incubation, the existing substances were removed, and the cells were washed once with DMEM/F-12 and treated with a WST assay reagent (EZ-Cytox kit, DoGenBio) together with the medium. After 2 hours, absorbance at 450 nm was measured to determine cell viability of IPEC-J2 cells. Cell viability of IPEC-J2 cells at each sample concentration are as shown in FIG. 3 and Table 5.

**[Table 5]**

| Sample Concentration (µM) | Cell Viability (%) | | |
|---|---|---|---|
| | DON Control | DON + Inactive | DON + GhSPG-216 |
| 0 | 100.0 | 100.0 | 100.00 |
| 0.5 | 79.0 | 82.3 | 105.9 |
| 1 | 71.4 | 71.2 | 106.4 |
| 2 | 64.6 | 64.9 | 99.5 |

Thus, upon treatment with the DON control and the DON + inactivated GhSPG-216 samples, cell viability significantly decreased to approximately 65% at 0.5 µM to 2 µM, whereas upon treatment with the DON + active GhSPG-216 sample, it was confirmed that cell viability was maintained at 100% or more. As set forth above, those skilled in the art will be able to understand that the present disclosure may be embodied in other specific forms without departing from the technical spirit or essential characteristics thereof. Therefore, the foregoing embodiments should be construed as being exemplified and not limiting the present disclosure. It should be understood that all changes or modifications derived from the definitions and the scopes of the claims and their equivalents fall within the scope of the present disclosure.

## Claims

1. A modified polypeptide having deoxynivalenol (DON) degradation activity, wherein the modified polypeptide is:
1) a polypeptide having an amino acid sequence of SEQ ID NO: 3; or
2) a polypeptide in which an amino acid at a position corresponding to position 65 of SEQ ID NO: 3 is substituted with a different amino acid.

2. The modified polypeptide according to claim 1, wherein the amino acid at the position corresponding to position 65 of SEQ ID NO: 3 is substituted with methionine (M).

3. The modified polypeptide according to claim 1, wherein the modified polypeptide has one or more improved properties selected from thermal tolerance and thermal stability compared to a polypeptide consisting of the amino acid sequence of SEQ ID NO: 1.

4. The modified polypeptide according to claim 2, wherein the polypeptide comprises an amino acid sequence of SEQ ID NO: 5.

5. A polynucleotide encoding the modified polypeptide of any one of claims 1 to 4.

6. A microorganism comprising one or more of: the modified polypeptide of claim 1; a polynucleotide encoding the modified polypeptide; and a vector comprising the polynucleotide.

7. A composition for degrading deoxynivalenol (DON), comprising one or more of: the modified polypeptide of claim 1; and a microorganism comprising one or more of the modified polypeptide, a polynucleotide encoding the modified polypeptide, and a vector comprising the polynucleotide.

8. A feed additive composition comprising one or more of: the modified polypeptide of claim 1; and a microorganism comprising one or more of the modified polypeptide, a polynucleotide encoding the modified polypeptide, and a vector comprising the polynucleotide.

9. A method for degrading deoxynivalenol (DON), comprising reacting deoxynivalenol (DON) with one or more of: the modified polypeptide of claim 1; and a microorganism comprising one or more of the modified polypeptide, a polynucleotide encoding the modified polypeptide, and a vector comprising the polynucleotide.

10. A method for preparing a feed product, comprising mixing a feed ingredient with a feed additive composition comprising one or more of: the modified polypeptide of claim 1; and a microorganism comprising one or more of the modified polypeptide, a polynucleotide encoding the modified polypeptide, and a vector comprising the polynucleotide.

11. A method for preparing a modified polypeptide having deoxynivalenol (DON) degradation activity, comprising culturing a microorganism comprising one or more of: the modified polypeptide of claim 1, a polynucleotide encoding the modified polypeptide, and a vector comprising the polynucleotide.

12. Use of one or more of: the modified polypeptide of claim 1; a microorganism comprising one or more of the modified polypeptide, a polynucleotide encoding the modified polypeptide, and a vector comprising the polynucleotide, for degrading deoxynivalenol (DON).
